# FASCICULE DE BREVET EUROPEEN

(11) **EP 0 253 690 B1**
(45) Date de publication et mention de la délivrance du brevet: **11.08.1993**
(21) Numéro de dépôt: 87401301.4
(22) Date de dépôt: 10.06.1987
(51) Int. Cl.: C12N 15/15, A61K 37/64

(54) **Variants de l'alpha 1-antitrypsine utiles notamment comme inhibiteurs de la kallikreine**
Alpha-1-antitrypsin-Varianten, besonders verwendbar als Kallikrein-Inhibitoren
Alpha-1-antitrypsin variants particularly useful as kallikrein inhibitors

(30) Priorité: 10.06.1986 FR 8608386
(43) Date de publication de la demande: 20.01.1988
(73) Titulaire: TRANSGENE S.A., 67082 Strasbourg Cédex (FR)
(72) Inventeur: Jallat, Sophie, F-67000 Strasbourg (FR); Courtney, Michael, F-67170 Brumath (FR)
(74) Mandataire: Warcoin, Jacques

(56) Documents cités:
- EP-A- 0 169 114
- J. CLIN. INVEST., vol. 76, décembre 1985, pages 635-637, The American Society for Clinical Investigation, Inc., US; M. SCHAPIRA et al.: "Recombinant Alpha1-antitrypsin Pittsburgh (Met358 - Arg) is a potent inhibitor of plasma kallikrein and activated factor XII fragment"
- THE JOURNAL OF BIOLOGICAL CHEMISTRY, vol. 260, no. 4, 25 février 1985, pages 2432-2436, The American Society of Biological Chemists, Inc., US; G.S. SALVESEN et al.: "Primary structure of the reactive site of human C1-inhibitor"
- SYMPOSIA BIOLOGICA HUNGARICA, vol. 25, 1984, pages 277-298; S. BAJUSZ: "Interaction of trypsin-like enzymes with small inhibitors"
- NATURE, vol. 313, no. 5998, 10 janvier 1985, pages 149-151, Londres, GB; M. COURTNEY et al.: "Synthesis in E. coli of Alpha1-antitrypsin variants of therapeutic potential for emphysema and thrombosis"
- CHEMICAL ABSTRACTS, vol. 102, 1985, page 431, résumé no. 93971e, Columbus, Ohio, US; V.H. DONALDSON et al.: "Variability in purified dysfunctional C1-inhibitor proteins from patients with hereditary angioneurotic edema. Functional and analytical gel studies"
- BIOLOGICAL ABSTRACTS, Piladelphia, US; M. SHAPIRA et al.: "Protection by alpha-1 antitrypsin-alanine-357-arginine-358 against arterial hypotension induced by factor XII fragment", & CLINICAL RESEARCH (US) 1987, vol. 35, no. 3, page 432A
- THE NEW ENGLAND JOURNAL OF MEDICINE, vol. 309, no. 12, 22 septembre 1983, pages 694-698; M.C. OWEN et al.: "Mutation of antitrypsin to antithrombin - alpha1-antitrypsin Pittsburgh (358-Met-Arg), A fatal bleeding disorder"
- PROTEIN ENGINEERING, vol. 1, no. 1, 1986, pages 29-35; S. JALLAT et al.: "Altered specificities of genetically engineered alpha1 antitrypsin variants"
- J. CELL. BIOCHEM. SUPPL., vol. 0, no. 10, part-A, 1986, page 274; S.JALLAT et al.: "Modeling alpha1-antitrypsin function by protein engineering", & Symposium on proteases in biological control and biotechnology held at the 15th Annual UCLA meeting on molecular and cellular biology, Los Angeles, California, US, 9-15 février 1986

## Description

La présente invention concerne un nouveau variant de l'alpha₁ -antitrypsine humaine.

Différents variants de l'alpha₁- antitrypsine humaine ont déjà été décrits, notamment dans la demande de brevet WO/86 00337.

La fonction principale de l'alpha₁- antitrypsine (alpha₁ -AT) est l'inhibition de l'élastase neutrophile dans les poumons.

L'alpha₁- AT ne présente aucun rôle in vivo dans la régulation de la protéase de coagulation, thrombine, pas plus que des protéases de contact de phase, facteur XII et kallikréine.

Toutefois, la substitution du radical Met en position P1 du centre réactif de (Met³⁵⁸)alpha-₁ AT par une arginine conduit à une inhibition efficace de ces protéases, ce qui met en évidence le rôle critique du radical en P1 dans l'inhibition spécifique.

On a pu mettre, en outre, en évidence que le radical en P2 (position 357 de l'alpha₁ -AT) est également important. Tout d'abord pour maintenir une conformation appropriée des centres réactifs et, ensuite, en influençant les propriétés d'inhibition.

Par exemple, l'antithrombine III (ATIII), qui est un inhibiteur spécifique de la thrombine in vivo, présente un radical Arg (site préféré de clivage pour la thrombine) en position P1 et un radical Gly en position P2. L'inhibiteur C1, qui est in inhibiteur prédominant de la kallikréine et du facteur XII, présente également un radical arginine en position P1 mais un radical Ala en position P2. L'inhibiteur C1 est, in vivo, relativement inefficace à l'égard de la thrombine, ce qui reflète peut-être une dimension de site actif plus petit dans le cas de cette enzyme.

L'objet de la présente invention est un nouveau variant de l'alpha₁-AT humaine dans lequel les sites P1 et P2 ont été modifiés de façon à tenter de limiter la dimension de la cible sur ce variant.

L'invention concerne un variant en position 357 de l'alpha₁₋AT humaine ou du (deltaGlu¹→ Gly⁵)alpha₁-AT.

En remplaçant la proline en position 357 par un radical Ala, on recrée le centre réactif de l'inhibiteur C1 et on dirige le variant inhibiteur vers la kallikréine au lieu de la thrombine.

C'est pourquoi la présente invention concerne plus particulièrement un nouveau variant (Ala³⁵⁷,Arg³⁵⁸)alpha₁-AT à titre d'inhibiteur de la kallikréine et qui peut être utilisé à titre de médicament comme cela sera décrit ci-après.

Mais, l'invention concerne également les variants (Ala³⁵⁷) des différents variants décrits dans le brevet WO 86 00337, en particulier, (Ala³⁵⁷) (deltaGlu¹→Gly⁵)alpha₁-AT, ainsi que les variants correspondants en position 358, (Gly³⁵⁸), (Ala³⁵⁸), (Ile³⁵⁸), (Val³⁵⁸), (Leu³⁵⁸) et (Phe³⁵⁸).

L'invention concerne aussi bien les variants sous leur forme glycosylée que non glycosylée.

La kallikréine est une protéase sérique qui est impliquée à la fois dans l'initiation de la coagulation et dans la fibrinolyse. Elle est également responsable du clivage, à partir du kininogène de la bradykinine, qui est un oligo-peptide très actif jouant un rôle essentiel dans différents processus, notamment inflammatoires, et dans la réponse à la douleur.

On a montré également que la bradykinine pourraît participer à la régulation de la pression sanguine.

L'inhibiteur C1 est le principal inhibiteur plasmatique de la kallikréine. Une déficience en cet inhibiteur conduit à une activation non régulée de la prékallikréine et du facteur XII qui conduit à un angio-oedème héréditaire.

Comme cela a été montré dans le brevet mentionné précédemment,l'(Arg³⁵⁸)alpha -AT est plus efficace que l'inhibiteur C1 dans l'inactivation à la fois de la kallikréine (activité 4,1 fois supérieure) et facteur XIIF (activité 11,5 fois plus élevée). C'est pourquoi la protéine (Arg³⁵⁸)alpha₁-AT semble avoir les caractéristiques d'un produit pour le traitement de l'angio-oedème héréditaire. Toutefois, cette molécule présente également une activité antithombinique très importante et il peut être intéressant, dans certains cas, d'avoir des variants différents possédant un mode d'action plus spécifique ; c'est ce qui est proposé dans le cadre de la présente invention.

Le nouveau variant(Ala³⁵⁷,Arg³⁵⁸)alpha₁- AT présente une activité inhibitrice de la kallikréine très améliorée en présentant, au contraire, des propriétés anti-thrombiniques très faibles.

Un tel produit peut être utilisé à titre de médicament inhibiteur de la kallikréine, par exemple pour une thérapie de remplacement des déficiences en inhibiteur C1. Il est également possible de prévoir son utilisation dans le traitement des chocs septiques et également pour la régulation de la pression sanguine et le traitement des hypertensions.

Les exemples ci-après sont destinés plus particulièrement à illustrer d'autres avantages et caractéristiques de la présente invention sans toutefois en limiter aucunement la portée.

### EXEMPLE 1

### EXPRESSION DE L'ALPHA 1-AT MODIFIEE DANS E. COLI

Les plasmides et matériaux génétiques utilisés dans ces constructions ont déjà été décrits dans la demande de brevet mentionnée précédemment.

### MUTAGENESE DU GENE HUMAIN DE L'ALPHA 1-AT

Le remplacement du résidu Met³⁵⁸ de alpha₁- AT par l'arginine et du résidu Pro³⁵⁷ par Ala a été effectué par mutagénèse directe à l'aide d'oligonucléotides.

Le cADN de l'alpha₁- AT cloné a été sous-cloné dans le vecteur M13 et l'ADN du phage monobrin a été utilisé comme modèle pour un oligonucléotide complémentaire synthétique contenant la modification souhaitée (5ʹ GATAGACCTAGCTATGGCC 3ʹ). Le second brin est synthétisé en utilisant la polymérase de Klenow, puis on effectue une transformation et les plaques contenant le gène muté sont identifiées par criblage à l'aide de l'oligonucléotide.

Les mutations sont confirmées par séquençage direct.
séquence d'origine 
Ala Ile Pro Met Ser Ile
GCC ATA CCC ATG TCT ATC
séquence mutée

Ala Ile Ala Arg Ser Ile
GCC ATA GCT AGG TCT ATC

Cette technologie a été décrite en détail dans le brevet mentionné précédemment.

Le fragment du variant alpha₁- AT est ensuite introduit dans le plasmide pTG983 comme cela a été décrit dans le brevet précédent.

On obtient ainsi un plasmide dénommé pTG1946 qui est identique au plasmide pTG983 à l'exception du gène alpha₁- AT qui a été modifié sur la position 357 et 358.

Dans ce qui suit les plasmides utilisés sont les suivants :
pTG983 (alpha₁- AT non modifiée)
pTG1900 (Arg³⁵⁸ alpha₁- AT)
pTG1946 (Ala³⁵⁷ Arg³⁵⁸ alpha₁- AT).

### EXPRESSION DE L'ALPHA1 -AT MODIFIEE DANS E. COLI

Le variant selon la présente invention,obtenu par fermentation de bactéries E. coli transformées par pTG1946, a été comparé avec pTG983 et pTG1900.

Des extraits soniqués clarifiés ont été préparés à partir de cultures induites de E. coli TGE900 contenant l'un des trois plasmides précédents.

Des aliquotes d'extraits sont testées pour leur capacité à inhiber l'élastase neutrophile humaine (cette technique ayant déjà été décrite).

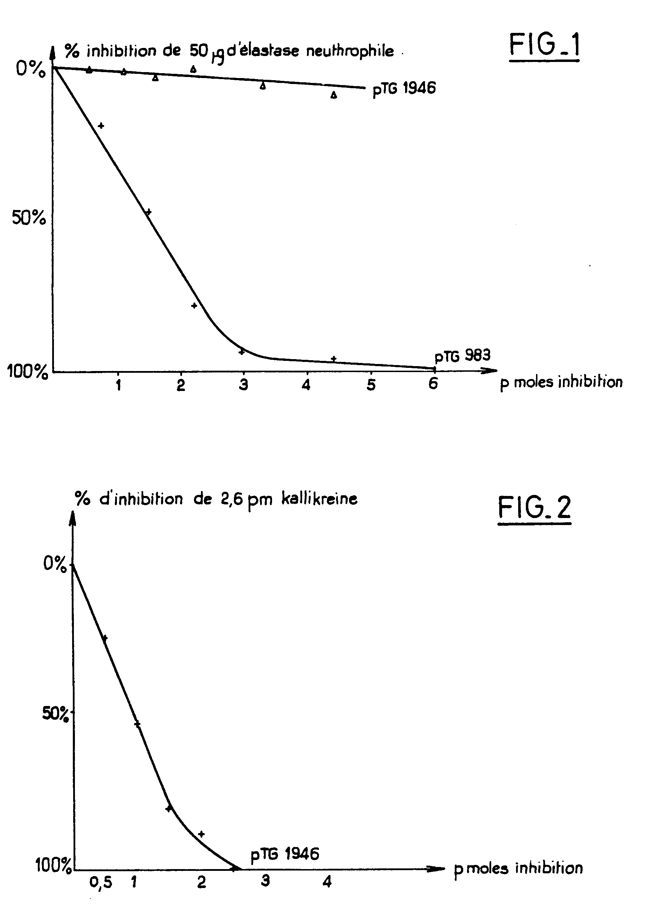

Les courbes d'inhibition représentées à la figure 1 montrent que,par rapport à l'alpha₁- AT normale (Met³⁵⁸ alpha₁- AT), le variant selon la présente invention (Ala³⁵⁷ Arg³⁵⁸)alpha₁- AT est incapable d'inhiber l'élastase neutrophile.

L'activité antikallikréine est évaluée en mesurant à 410 nm le taux résiduel d'hydrolyse de la chromosyme PK (benzoyl-Pro-Pre-Arg 4 nitroanilide-acétate, Boehringer Mannheim).

2,6 pmoles de kallikréine plasmatique humaine sont préincubées à 37°C avec une quantité croissante d'inhibiteur dans le tampon phosphate Na 0,1 M-NaCl 0,15 M. Après 30 minutes, la réaction est arrêtée en ajoutant 900 µmoles de substrat (concentration finale 0,5 mM) et l'activité enzymatique résiduelle est déterminée.

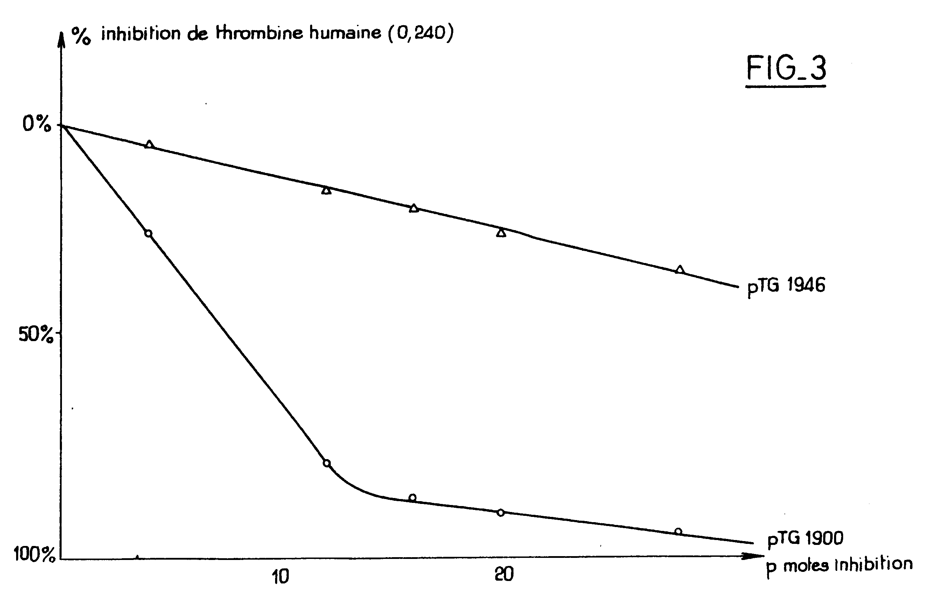

La figure 2 montre que le variant (Ala³⁵⁷, Arg³⁵⁸) alpha₁- AT est capable d'inhiber rapidement la kallikréine. Les courbes d'inhibition de thrombine (figure 3) et la comparaison avec l'alpha₁- AT(Arg³⁵⁸)indiquent que le variant(Ala³⁵⁷,Arg³⁵⁸)alpha₁- AT inhibe seulement très faiblement la thrombine humaine.

Le composé selon l'invention peut donc être utilisé dans une thérapie de substitution de l'inhibiteur C1

En utilisant la technique décrite précédemment et les procédés décrits dans le brevet WO 86 00337, on obtient les autres variants revendiqués.

La souche suivante a été déposée à la Collection Nationale de Cultures de Microorganismes de l'Institut Pasteur, 28 rue du Docteur-Roux, 75724 PARIS CEDEX 15 :
. souche E. coli TGE900 pTG1946
le 6 juin 1986 sous le n^{o} I-560.

## Revendications

1. Variant en position 357 de l'alpha₁-antitrypsine humaine ou du (deltaGlu¹→ Gly⁵)alpha₁-AT humaine.

2. Variant selon la revendication 1, caractérisé en ce qu'il s'agit d'un variant en position 357 et 358.

3. Variant selon l'une des revendications 1 et 2, caractérisé en ce qu'il s'agit d'un variant Ala³⁵⁷.

4. Variant de l'alpha₁-AT humaine de formule :
(Ala³⁵⁷,Arg³⁵⁸)alpha₁-AT.

5. Le variant selon la revendication 4 pour une utilisation comme inhibiteur de la kallikréine.

6. Un variant selon l'une des revendications 1 à 4 pour une utilisation comme médicament.

7. Le variant :
(Ala³⁵⁷,Arg³⁵⁸)alpha₁-AT
pour une utilisation comme médicament.

## Claims

1. A variant at position 357 of human alpha₁-antitrypsin or of human (deltaGlu¹→Gly⁵)alpha₁-AT.

2. The variant as claimed in claim 1, which is a variant at position 357 and 358.

3. The variant as claimed in one of claims 1 and 2, which is an Ala³⁵⁷ variant.

4. A variant of human alpha₁-AT of formula :
(Ala³⁵⁷,Arg³⁵⁸)alpha₁-AT.

5. The variant as claimed in claim 4 for a use as a kallikrein inhibitor.

6. A variant as claimed in one of claims 1 to 4 for a use as a medicinal product.

7. The variant :
(Ala³⁵⁷,Arg³⁵⁸)alpha₁-AT
for a use as a medicinal product.

## Patentansprüche

1. Variante in 357-Stellung von humanem alpha₁-Antitrypsin oder von humanem (deltaGlu¹→ Gly⁵)alpha₁-AT.

2. Variante nach Anspruch 1, dadurch gekennzeichnet, daß es sich um eine Variante in 357- und 358-Stellung handelt.

3. Variante nach einem der Ansprüche 1 und 2, dadurch gekennzeichnet, daß es sich um eine Ala³⁵⁷ -Variante handelt.

4. Variante von humanem alpha₁-AT der Formel:
(Ala³⁵⁷,Arg³⁵⁸)alpha₁-AT.

5. Variante nach Anspruch 4 zur Verwendung als Kallikrein-Inhibitor.

6. Variante nach einem der Ansprüche 1 bis 4 zur Verwendung als Arzneimittel.

7. Die Variante
(Ala³⁵⁷,Arg³⁵⁸)alpha₁-AT
zur Verwendung als Arzneimittel.
